# EUROPEAN PATENT SPECIFICATION

(11) **EP 1 059 913 B1**
(45) Date of publication and mention of the grant of the patent: **29.09.2004**
(21) Application number: 99908952.7
(22) Date of filing: 04.03.1999
(51) Int. Cl.: A61K 9/107, A61K 38/13, A61K 31/435, A61K 9/48

(54) **EMULSION PRECONCENTRATES CONTAINING CYCLOSPORIN OR A MACROLIDE**
CYCLOSPORIN ODER MACROLIDE ENTHALTENDE EMULSIONSVORKONZENTRATE
PRECONCENTRES D'EMULSION A BASE DE CYCLOSPORINE OU D'UN MACROLIDE

(30) Priority: 06.03.1998 GB 9804742; 10.03.1998 GB 9805104; 11.03.1998 GB 9805199
(43) Date of publication of application: 20.12.2000
(60) Divisional application: 03013016.5
(73) Proprietor: Novartis AG, 4056 Basel (CH); Novartis Pharma GmbH, 1230 Wien (AT)
(72) Inventor: AMBÜHL, Michael, CH-4313 Möhlin (CH); LÜCKEL, Barbara, D-79540 Lörrach (DE); RICHTER, Friedrich, D-67157 Wachenheim (DE); HÄBERLIN, Barbara, CH-4125 Riehen (CH); MEINZER, Armin, D-79426 Buggingen (DE)
(74) Representative: Grubb, Philip William
(86) International application number: PCT/EP1999/001415
(87) International publication number: WO 1999/044584

(56) References cited:
- EP-A- 0 793 966
- EP-A- 1 092 429
- EP-A- 1 147 766
- WO-A-96/13273
- WO-A-97/36610
- WO-A-99/00002
- WO-A-99/29316
- FR-A- 2 636 534
- GB-A- 2 278 780
- US-A- 5 639 724

## Description

The present invention relates to novel galenic compositions, in particular novel galenic compositions in which the active ingredient is a difficultly soluble active agent e.g. a macrolide, or in particular a cyclic poly-N-methylated undecapeptide, or a cyclosporin. Cyclosporins also include peptolide variants. See e.g. GB patent publications nos. 2 222 770 and 2 257 359 A and equivalents world-wide.

As discussed in the said GB patent publications, the cyclosporins present highly specific difficulties in relation to administration generally and galenic composition in particular, including in particular problems of stability, drug bioavailability, and variability in inter- and intra-patient dose response .

In order to meet these and related difficulties, in GB patent publication no. 2 222 770 and 2 257 359 A, galenic compositions are disclosed comprising a cyclosporin as active ingredient and which take the form of, inter alia, an emulsion, e.g. microemulsion, or emulsion, e.g. microemulsion, pre-concentrate. Such compositions typically comprise 1) a hydrophilic component, 2) a lipophilic component, and 3) a surfactant.

(Micro-) Emulsion preconcentrates comprising a macrolide or cyclosporin are known from US 5,639,724, GB 2 278 780, WO 97 36 610, FR 2 636 534, WO 96 13 273 and WO 99 29 316.

In accordance with the present invention it has now surprisingly been found that particularly stable emulsion, e.g. microemulsion, or emulsion, e.g. microemulsion, pre-concentrate galenic compositions with difficultly soluble active agents having particularly interesting bioavailability characteristics and reduced variability in inter- and intra-subject bioavailability parameters, are obtainable using at least one component selected from the group consisting of (i) glyceryl di fatty acid ester, (ii) glyceryl mono fatty acid ester, (iii) a mixture of mono-, diglycerides of fatty acids, (iv) propylene glycol mono fatty acid ester, (v) fatty alcohols.

In accordance with the present invention, it has surprisingly been found that such emulsion, e.g. microemulsion, systems can, in contrast to the teaching of the art, in practice indeed be prepared comprising from any of the components specified above as a second component.

The present invention provides in one aspect a pharmaceutical composition which is an emulsion, e.g. microemulsion, pre-concentrate comprising
1) a difficultly soluble active agent, and a carrier medium comprising
2) a second component,
3) a lipophilic component, and
4) a surfactant.

The second component is for example:
i) glyceryl di fatty acid ester, e.g. C₆-C₁₆, e.g. C₈-C₁₀, e.g. C₈, e.g. Sunfat® GDC-N, and/or
ii) glyceryl mono fatty acid ester, e.g. C₆-C₁₄, e.g. C₈-C₁₀, e.g. Imwitor® 308, or Imwitor® 310, and/or
iii) a mixture of mono-, diglycerides of fatty acids, e.g. C₆-C₁₆, e.g. C₈-C₁₀, e.g. Imwitor® 742, or Capmul® MCM, and/or
iv) propylene glycol mono fatty acid ester, e.g. C₆-C₁₂, e.g. C₈-C₁₂, e.g. Lauroglycol® 90, Sefsol® 218, or Capryol® 90, and/or
v) fatty alcohols, e.g. C₆-C₂₀, saturated or mono-or di- unsaturated, e.g. oleyl alcohol, tetradecanol, dodecanol, decanol, and/or

Accordingly, the present invention provides in one aspect a composition in form of an emulsion or microemulsion pre-concentrate for oral administration comprising
1) a cyclosporine or macrolide, and a carrier medium comprising
2) a second component selected from the group consisting of
   (i) glyceryl di C₆-C₁₆ fatty acid ester,
   (ii) glyceryl mono C₆-C₁₄ fatty acid ester,
   (iii) a mixture of mono-, diglycerides of C₆-C₁₆ fatty acids,
   (iv) propylene glycol mono C₆-C₁₂ fatty acid ester,
   (v) fatty alcohols,
3) a lipophilic component, and
4) a surfactant,
with the proviso that when component 2)
consists of a mixture of mono-, diglycerides of C₈-C₁₀ fatty acids, said composition is free of a C₆-C₁₂ fatty acid triglyceride.

In accordance with the present invention, it has surprisingly been found that a cylosporine or macrolide has a high solubility, e.g. a solubility of from about 20 to about 50%, in the second component of the present invention. For example the solubility of a cyclosporin or a macrolide in Sunfat®GDC-N about 33%, in Lauroglycol®90 about 40%, in Dodecanol about 37.5%, in Tetradecanol about 37.5%, in Sefsol®218 about 50%, in oleyl alcohol more than 20%.

Accordingly, the present invention provides in one aspect a composition in form of an emulsion or microemulsion pre-concentrate for oral administration comprising
1) a cyclosporine or macrolide, and a carrier medium comprising
2) a second component wherein component I) has a solubility of from about 20 to about 50%
3) a lipophilic component, and
4) a surfactant.

Preferably the composition is in the form of an "emulsion, e.g. microemulsion, preconcentrate" of the type providing o/w (oil-in-water) emulsions, e.g. microemulsions. However the composition may be in the form of an emulsion, e.g. microemulsion, which additionally contains an aqueous component; preferably water.

An "emulsion, e.g. microemulsion, preconcentrate" is defined in this specification as being a composition which spontaneously forms an emulsion, e.g. microemulsion, in an aqueous medium, for example, in water, for example on dilution of 1:1 to 1:10, e.g. 1:10, or in the gastric juices after oral application.

A microemulsion is thermodynamically stable and contains dispersed particles of a mean size less than about 200 nm. Generally microemulsions comprise droplets or particles having a mean diameter of less than about 150 nm; typically less than 100 nm, generally greater than 10nm, and stable over periods in excess of 24 hours. A "microemulsion" may be a non-opaque or substantially non-opaque, alternatively it may be a translucent colloidal dispersion that is formed spontaneously or substantially spontaneously when its components are brought into contact. Further characteristics can be found in the above mentioned British patent application 2 222 770.

In one aspect the present invention provides a composition according to the present invention, the relative proportion of the cyclosporine or macrolide, the second component, the lipophilic component and the surfactant in said composition being such that upon dilution with water to a ratio of 1 part by weight of said composition to 1 to 10 parts by weight of water, an oil-in-water microemulsion having particles of a mean size of less than 200 nm, is spontaneously formed.

In a further alternative aspect the lipophilic component may comprise 5 to 85 % by weight of the carrier medium, e.g. 10 to 85%; preferably 15 to 70 % by weight, more preferably 20 to 60 % by weight and even more preferably about 25 % by weight.

In a further alternative aspect the surfactant may comprise 5 to 80 % by weight of the carrier medium; preferably 10 to 70 % by weight, more preferably 20 to 60 % by weight and even more preferably about 40 % by weight.

In a further alternative aspect the second component may comprise 5 to 50 % by weight of the carrier medium, e.g. 10 to 50%; preferably 15 to 40 % by weight, more preferably 20 to 35 % by weight.

In one aspect the present invention provides a composition according to the present invention comprising the second component in an amount of 5 to 50 %, the lipophilic component in an amount of 5 to 85 %, and the surfactant in an amount of 5 to 80 % by weight of the carrier medium.

The active agent may be present in an amount by weight of up to about 20% by weight of the composition. The active agent is preferably present in an amount of 1 to 15 % by weight of the composition, for example about 2 to 10 %.

In one aspect the present invention provides a composition according to the present invention comprising the cyclosporine or macrolide in an amount of 1 to 15% by weight of the composition.

The difficultly soluble active agent preferably is a lipophilic drug, e.g. a cyclosporin or a macrolide. The term "difficultly soluble", as used herein, is understood to mean a solubility in water at 20°C of less than 1 e.g. 0.01 % weight/volume.

Cyclosporins to which the present invention applies are any of those having pharmaceutical utility, e.g. as immunosuppressive agents, anti-parasitic agents and agents for the reversal of multi-drug resistance, as known and described in the art, in particular Cyclosporin A (also known as and referred to hereinafter as Ciclosporin), Cyclosporin G, [0-(2-hydroxyethyl)-(D)Ser]⁸-Ciclosporin, and [3'-deshydroxy-3'-keto-MeBmt]¹-[Val]²-Ciclosporin. Ciclosporin is preferred.

In one aspect the present invention provides a composition according to the present invention wherein the cyclosporine is Cyclosporin A.

The term "macrolide" as used herein, refers to a macrocyclic lactone, for example a compound having a 12- membered or larger lactone ring. Of particular interest are the "lactam macrolides", i.e., macrocyclic compounds having a lactam (amide) bond in the macrocycle in addition to a lactone (ester) bond, for example the lactam macrolides produced by microorganisms of the genus Streptomyces such as rapamycin, ascomycin, and FK-506, and their numerous derivatives and analogues. Such lactam macrolides have been shown to have interesting pharmaceutical properties, particularly immunosuppressive and anti-inflammatory properties.

Rapamycin is an immunosuppressive lactam macrolide that is produced by Streptomyces hygroscopicus. The structure of rapamycin is given in Kesseler, H., et al.; 1993; Helv. Chim. Acta; 76: 117. See, e.g., McAlpine, J.B., et al., J. Antibiotics (1991) 44: 688; Schreiber, S.L., et al., J. Am. Chem. Soc. (1991) 113: 7433; US Patent No. 3 929 992.

Rapamycin is an extremely potent immunosuppressant and has also been shown to have antitumor and antifungal activity. Its utility as a pharmaceutical, however, is restricted by its very low and variable bioavailability as well as its high toxicity. Moreover, rapamycin is highly insoluble, making it difficult to formulate stable galenic compositions. Numerous derivatives of rapamycin are known. Certain 16-O-substituted rapamycins are disclosed in WO 94/02136. 40-O-substituted rapamycins are described in, e.g., in US 5 258 389 and WO 94/09010 (O-aryl and O-alkyl rapamycins); WO 92/05179 (carboxylic acid esters), US 5 118 677 (amide esters), US 5 118 678 (carbamates), US 5 100 883 (fluorinated esters), US 5 151 413 (acetals), US 5 120 842 (silyl ethers), WO 93/11130 (methylene rapamycin and derivatives), WO 94/02136 (methoxy derivatives), WO 94/02385 and WO 95/14023 (alkenyl derivatives). 32-O-dihydro or substituted rapamycins are described, e.g., in US 5 256 790.

Rapamycin and its structurally similar analogues and derivatives are termed collectively as "rapamycins".

Ascomycins, of which FK-506 and ascomycin are the best known, comprise another class of lactam macrolides, many of which have potent immunosuppressive and anti-inflammatory activity. FK506 is a lactam macrolide immunosuppressant that is produced by Streptomyces tsukubaensis No 9993. The structure of FK506 is given in the appendix to the Merck Index, 11th ed. (1989) as item A5. Ascomycin is described, e.g., in US patent 3,244,592. Many derivatives of ascomycin and FK-506 have been synthesized, including halogenated derivatives such as 33-epi-chloro-33-desoxy-ascomycin described in EP 427 680. Ascomycin, FK-506 and their structurally similar analogues and derivatives are termed collectively "ascomycins".

The macrolide may, therefore, be rapamycin or an O-substituted derivative in which the hydroxyl group on the cyclohexyl ring of rapamycin is replaced by -OR₁ in which R₁ is hydroxyalkyl, hydroalkoxyalkyl, acylaminoalkyl and aminoalkyl; for example 40-O-(2-hydroxy)ethyl-rapamycin, 40-O-(3-hydroxy)propyl-rapamycin, 40-O-[2-(2-hydroxy)ethoxy]ethyl-rapamycin and 40-O-(2-acetaminoethyl)-rapamycin.

A preferred compound is 40-0-(2-hydroxy)ethyl rapamycin as disclosed in WO 94/09010.

Examples of compounds of the FK 506 class are those mentioned above. They include for example FK 506, ascomycin and other naturally occurring compounds. They include also synthetic analogues.

A preferred compound of the FK 506 class is disclosed in EP 427 680, e.g. Example 66a also known as 33-epi-chloro-33-desoxy-ascomycin. Other preferred compounds are disclosed in EP 465 426, and in EP 569 337, e.g. the compound of Example 71 in EP 569 337.

The second component may be any of components i) to v) individually or in combination with one, two or more of the other components i) to v). Examples of suitable second components for use in this invention are:
i) glyceryl di C₆-C₁₆ fatty acid ester. Diglycerides suitable for use in the compositions of the invention include both symmetric (i.e. α,α¹-diglycerides) as well as assymetric diglycerides (i.e. α,β-diglycerides) and acetylated derivatives thereof. They also include both uniform glycerides (in which the fatty acid constituent is composed primarily of a single fatty acid) as well as mixed glycerides (i.e. in which the fatty acid constituent is composed of various fatty acids) any acetylated derivatives thereof. The fatty acid constituent may include both saturated and unsaturated fatty acids having a chain length of from e.g. C₆-C₁₆, e.g. C₈-C₁₀, e.g. C₈. Particularly suitable is caprylic diglyceride which is commercially available, e.g. under the trade name Sunfat® GDC-N, e.g, from Taiyo Kagaku Co., Ltd. Sunfat® GDC-N has an acid value of about 0.3, a diglyceride content of about 78.8%, and a monoester content of about 8.9%.
ii) glyceryl mono C₆-C₁₄ fatty acid ester. They may be obtainable by esterification of glycerol with vegetabel oil followed by molecular distillation. Monoglycerides suitable for use in the compositions of the invention include both symmetric (i.e. β-monoglycerides) as well as assymetric monoglycerides (α-monoglycerides) and acetylated derivatives thereof. They also include both uniform glycerides (in which the fatty acid constituent is composed primarily of a single fatty acid) as well as mixed glycerides (i.e. in which the fatty acid constituent is composed of various fatty acids) any acetylated derivatives thereof. The fatty acid constituent may include both saturated and unsaturated fatty acids having a chain length of from e.g. C₈-C₁₀. Particularly suitable are caprylic or capric acid monoglycerides which are commercially available, e.g. under the trade names Imwitor® 308 or Imwitor® 310, respectively, from e.g. Condea. For example Imwitor® 308 comprises at least 80 % monoglycerides and exhibits the following additional characterising data: free glycerol max 6 %, acid value max. 3, saponification value 245-265, iodine value max. 1, water content max. 1 %. Typically it comprises 1 % free glycerol, 90 % monoglycerides, 7 % diglycerides, 1 % triglycerides (H. Fiedler, loc cit, vol 1, page 798).
iii) a mixture of mono-, diglycerides of C₆-C₁₆ fatty acids. Mixed mono-, di-glycerides suitable for use in the compositions of the invention include both symmetric (i.e. β-monoglycerides and α,α¹-diglycerides) as well as assymetric mono- and di-glycerides (i.e. α-monoglycerides and α,β-diglycerides) and acetylated derivatives thereof. They also include both uniform glycerides (in which the fatty acid constituent is composed primarily of a single fatty acid) as well as mixed glycerides (i.e. in which the fatty acid constituent is composed of various fatty acids) any acetylated derivatives thereof. The fatty acid constituent may include both saturated and unsaturated fatty acids having a chain length of from e.g. C₈-C₁₀. Particularly suitable are mixed caprylic and capric acid mono- and di-glycerides as commercially available, e.g. under the trade name Imwitor® 742, from e.g. Condea. For example Imwitor® 742 comprises at least 45% monoglycerides and exhibits the following additional characterising data: free glycerol max. 2 %, acid value max. 2, saponification value 250-280, iodine value max. 1, water max. 2 % (H. Fiedler, loc cit, vol 1, page 798). Further suitable is a mono/diglyceride of caprylic/capric acid in glycerol as known and commercially available under e.g. the trade name Capmul® MCM from e.g. Abitec Corporation. Capmul® MCM exhibits the following additional characterising data: acid value 2.5 max., alpha-Mono (as oleate) 80% min., free glycerol 2.5% max., iodine value 1 max., chain length distribution: caproic acid (C6) 3% max., caprylic acid (C8) 75% min., capric acid (C10) 10% min., lauric acid (C12) 1.5% max., moisture (by Karl Fisher) 0.5% max. (manufacturer information).
iv) propylene glycol mono C₆-C₁₂ fatty acid ester. The fatty acid constituent may include both saturated and unsaturated fatty acids having a chain length of from e.g. C₈-C₁₂. Particularly suitable are propylene glycol mono ester of caprylic and lauric acid as commercially available, e.g. under the trade names Sefsol® 218, Capryol®90 or Lauroglycol®90, from e.g. Nikko Chemicals Co., Ltd. or Gattefossé. For example Lauroglycol®90 exhibits the following additional characterising data: acid value max. 8, saponification value 200-220, iodine value max. 5, free propylene glycol content max. 5%, monoester content min. 90%; Sefsol® 218 exhibits the following additional characterising data: acid value max. 5, hydroxy value 220-280 (H. Fiedler, loc cit, vol 2, page 906, manufacturer information).
v) fatty alcohols. The fatty alcohol constituent may include both saturated and mono- or di-unsaturated fatty alcohols having a chain length of from e.g. C₆-C₂₀. Particularly suitable are, e.g. oleyl alcohol, tetradecanol, dodecanol, or decanol. For example oleyl alcohol is commercially available under the trade mark HD-Eutanol® V from e.g. Henkel KGaA. Oleyl alcohol exhibits the following additional characterising data: acid value max 0.1, hydroxy value of about 210, iodine value of about 95, saponification vaiue max 1, D.²⁰ about 0,849, n_{D}²⁰ 1,462, molecular weight 268, viscosity (20°) about 35 mPa s (manufacturer information).

Although any pharmaceutically acceptable components selected from the group specified above may be used in the composition of the invention, certain components are preferred.

Accordingly, the present invention provides in one aspect a composition according to the present invention, wherein the second component is selected from fatty alcohols.

The second component may also comprise a co-component which may be hydrophilic, e.g. selected from Transcutol (which has the formula C₂H₅-[O-(CH₂)₂]₂-OH), Glycofurol (also known as tetrahydrofurfuryl alcohol polyethylene glycol ether) and 1,2-propylene glycol. The second component may include further hydrophilic co-components, for example lower alkanols such as ethanol. These co-components will generally be present in partial replacement of other components of the second component. While the use of ethanol in the compositions is not essential, it has been found to be of particular advantage when the compositions are to be manufactured in soft gelatine, encapsulated form. This is because storage characteristics are improved, in particular the risk of active agent precipitation following encapsulation procedures is reduced. Thus the shelf life stability may be extended by employing ethanol or some other such co-component as an additional ingredient of the second component. In a further alternative aspect the ethanol may comprise 0 to 60 % by weight of the second component; preferably 20 to about 55% by weight and more preferably about 40 to 50 % by weight. Small quantities of liquid polyethylene glycols may also be included in the second component.

GB 2 222 770 A discloses a wide variety of lipophilic components suitable for use in the present invention. Typical examples for lipophilic components are:
(i) medium chain fatty acid triglycerides, e.g. C₆-C₁₂, e.g. Miglyol® 812, and/or
(ii) mixed mono-, di-, tri-glycerides, e.g. C₆-C₂₀, e.g. C₁₆-C₁₈, e.g. Maisine®, and/or
(iii) transesterified ethoxylated vegetable oils, e.g. Labrafil®, and/or
(iv) propylene glycol mono fatty acid esters, e.g. C₁₄-C₁₈, e.g. propylene glycol hydroxystearate, propylene glycol isostearate, propylene glycol ricinoleate, propylene glycol stearate, and/or
(v) propylene glycol di fatty acid esters, e.g. C₆-C₂₀, e.g. C₈-C₁₂, e.g. propylene glycol dicaprylate, e.g. Miglyol® 840, or propylene glycol dilaurate, and/or
(vi) esterified compounds of fatty acid and primary alcohol, e.g. C₈-C₂₀ fatty acids and C₂-C₃ alcohols, e.g. ethyl linoleate, and/or
(vii) mono- and/or di-glyceride, e.g. a mixture of mono- and di-glycerides with e.g. a monoglyceride of C₁₈ fatty acid as its main component, e.g. GMOrphic®-80 or Tegin® O.

Preferred lipophilic components are medium chain fatty acid triglycerides, mixed mono-, di-, tri-glycerides, and transesterified ethoxylated vegetable oils.

Accordingly, in one aspect the present invention provides a composition according to the present invention wherein the lipophilic component is selected from the group consisting of (i) medium chain fatty acid triglycerides, (ii) mixed mono-, di-, tri-glycerides, and (iii) transesterified ethoxylated vegetable oils.

In another aspect the lipophilic component may comprise a medium chain triglyceride and/or a mono-and di-glyceride or a mixture thereof.

As the medium chain fatty acid triglyceride in the lipophilic component a triglyceride of saturated fatty acid having 6 to 12, e.g. 8 to 10, carbon atoms can be used. Suitable medium chain fatty acid triglycerides are those known and commercially available under the trade names Acomed®, Myritol®, Captex®, Neobee®M 5 F, Miglyol®810, Miglyol®812, Miglyol®818, Mazol®, Sefsol®860, Sefsol®870; Miglyol®812 being the most preferred. Miglyol®812 is a fractionated coconut oil comprising caprylic-capric acid triglycerides and having a molecular weight of about 520 daltons. Fatty acid composition = C₆ max. about 3%, C₈ about 50 to 65%, C₁₀ about 30 to 45%, C₁₂ max 5%; acid value about 0.1; saponification value about 330 to 345; iodine value max 1. Miglyol® 812 is available from Condea. Neobee® M 5 F is a fractionated caprylic-capric acid triglyceride available from coconut oil; acid value max. 0.2; saponification value about 335 to 360; iodine value max 0.5, water content max. 0,15%, D.²⁰ 0,930-0,960, n_{D}²⁰ 1,448-1,451 (manufacturer information). Neobee® M 5 F is available from Stepan Europe.

These triglycerides are described in Fiedler, H. P., loc cit, the contents of which are hereby incorporated by reference.

In a further alternative aspect preferably monoglycerides comprise from about 25 to about 50%, based on the total weight of the lipophilic components. More preferably from about 30 to about 40% (for example 35 to 40%) monoglycerides are present.

In a further alternative aspect preferably diglycerides comprise from about 30 to about 60%, based on the total weight of the lipophilic component. More preferably from about 40 to about 55% (for example 48 to 50%) diglycerides are present.

In a further alternative aspect triglycerides suitably comprise at least 5% but less than about 25 %, based on the total weight of the lipophilic component. More preferably from about 7.5 to about 20% (for example from about 9 to 12%) triglycerides are present.

Suitable mixed mono-, di-, tri-glycerides are those known and commercially available under the trade name Maisine® from Gattefossé. They are transesterification products of corn oil and glycerol. Such products are comprised predominantly of linoleic and oleic acid mono-, di- and tri-glycerides together with minor amounts of palmitic and stearic acid mono-, di- and tri-glycerides (corn oil itself being comprised of ca. 56% by weight linoleic acid, 30% oleic acid, ca. 10% palmitic and ca. 3% stearic acid constituents). Physical characteristics are: free glycerol max 10%, monoglycerides ca. 40%, diglycerides ca. 40%, triglycerides ca. 10%, free oleic acid content ca. 1%. Further physical characteristics are: acid value max. 2, iodine value of 85-105, saponification value of 150-175, mineral acid content = 0. The fatty acid content for Maisine® is typically: palmitic acid ca. 11%, stearic acid ca. 2.5%, oleic acid ca. 29%, linoleic acid ca. 56%, others ca. 1.5% (H. Fiedler, loc cit, vol 2, page 958; manufacturer information).

In a further alternative aspect the lipophilic component may alternatively comprise e.g. a pharmaceutically acceptable oil, preferably with an unsaturated component such as a vegetable oil or fish oil.

The lipophilic component may alternatively comprise suitable transesterified ethoxylated vegetable oils such as those obtained by reacting various natural vegetable oils (for example, maize oil, kernel oil, almond oil, ground nut oil, olive oil, soybean oil, sunflower oil, safflower oil and palm oil, or mixtures thereof) with polyethylene glycols that have an average molecular weight of from 200 to 800, in the presence of an appropriate catalyst. These procedures are known and an example is described in US Patent 3 288 824. Transesterified ethoxylated corn oil is particularly preferred.

Transesterified ethoxylated vegetable oils are known and are commercially available under the trade name Labrafil® (H. Fiedler, loc cit, vol 2, page 880). Examples are Labrafil® M 2125 CS (obtained from corn oil and having an acid value of less than about 2, a saponification value of 155 to 175, an HLB value of 3 to 4, and an iodine value of 90 to 110), and Labrafil® M 1944 CS (obtained from kernel oil and having an acid value of about 2, a saponification value of 145 to 175 and an iodine value of 60 to 90). Labrafil® M 2130 CS (which is a transesterification product of a C₁₂₋₁₈ glyceride and polyethylene glycol and which has a melting point of about 35 to 40°C, an acid value of less than about 2, a saponification value of 185 to 200 and an iodine value of less than about 3) may also be used. The preferred transesterified ethoxylated vegetable oil is Labrafil® M 2125 CS which can be obtained, for example, from Gattefossé, Saint-Priest Cedex, France.

Further suitable lipophilic components for use in this invention are, e.g. propylene glycol mono- and di-fatty acid esters such as propylene glycol dicaprylate (also known and commercially available under the trade name Miglyol® 840 from e.g. Condea; H. Fiedler, loc cit, vol 2, page 1008) or propylene glycol dilaurate, propylene glycol hydroxystearate, propylene glycol isostearate, propylene glycol laurate, propylene glycol ricinoleate, propylene glycol stearate and so forth (Fiedler, loc. cit., 2, p. 1277 ff).

As another lipophilic component esterified compounds of fatty acid and primary alcohol may be used. They may include esterified compounds of fatty acid having 8 to 20 carbon atoms and primary alcohol having 2 to 3 carbon atoms, for example, isopropyl myristate, isopropyl palmitate, ethyl linoleate, ethyl oleate, etc., with an esterified compound of linoleic acid and ethanol being particularly preferable.

In a further aspect as the lipophilic component a mono-and/or di-glyceride may be used. A mono- and/or di-glyceride is e.g. a mixture of glycerol mono- and di-esters of a fatty acid in which the monoglyceride content is e.g. at least 40%. It is preferable that the mono- and di-glycerides contain a monoglyceride of C18 fatty acid as its main component. Such a compound has been commercialized under the trade name GMO®AV1 (Croda Co.), ATMOS®300 (ICI Co.), GMOrphic®-80 (Eastman Co.), Tegin®O (Goldschmidt Co.), etc. For example GMOrphic®-80 (glyceryl monooleate) exhibits the following additional characterising data: monoglyceride content min. 94%, C18:1 content 75% min., peroxide value max. 2.5, C18:2 + C18:3 max. 15%, C16:0 + C18:0 + C20:0 max. 10%, water max. 2%, acid value max. 3, iodine value 65-75, saponification value 155-165, free glycerine max. 1%, hydroxyl number 300-330 (manufacturer information). Tegin® O (glyceryl oleate) exhibits the following additional characterising data: monoglyceride content 55-65%, peroxide value max. 10, water content max. 1%, acid value max. 2, iodine value 70-76, saponification value 158-175, free glycerol max. 2%, (manufacturer information).

Typically the mono/diglyceride is present at a concentration of 5-10% by weight, e.g. of the composition, of the carrier medium or of the lipophilic component.

In a further alternative aspect in the composition of the present invention, any one of the above lipophilic components, e.g. oils, alone or in combination may be used as the lipophilic component. When a mixture of any of the medium chain triglyceride and mono- and di-glyceride is used in the lipophilic component, they may be present in the mixing ratio of 1:0.1-1, preferably 1:0.1-0.5, on the basis of weight.

In the pharmaceutical composition of the present invention, in a further alternative aspect the constitutional ratio of the lipophilic component to cyclosporin is preferably 1-10:1 and more preferably 2-6:1, on the basis of weight.

Examples of suitable surfactants for use in this invention are:
i) reaction products of a natural or hydrogenated castor oil and ethylene oxide. The natural or hydrogenated castor oil may be reacted with ethylene oxide in a molar ratio of from about 1:35 to about 1:60, with optional removal of the polyethyleneglycol component from the products. Various such surfactants are commercially available. The polyethyleneglycol-hydrogenated castor oils available under the trade name Cremophor® are especially suitable. Particularly suitable are Cremophor® RH 40, which has a saponification value of about 50 to 60, an acid value less than about 1, a water content ( Fischer) less than about 2%, an n_{D}⁶⁰ of about 1.453 to 1.457 and an HLB of about 14 to 16; and Cremophor® RH 60, which has a saponification value of about 40 to 50, an acid value less than about 1, an iodine value of less than about 1, a water content (Fischer) of about 4.5 to 5.5%, an n_{D}⁶⁰ of about 1.453 to 1.457 and an HLB of about 15 to 17. An especially preferred product of this class is Cremophor® RH40. Also suitable are polyethyleneglycol castor oils such as that available under the trade name Cremophor® EL, which has a molecular weight (by steam osmometry) of about 1630, a saponification value of about 65 to 70, an acid value of about 2, an iodine value of about 28 to 32 and an n_{D}²⁵ of about 1.471.
   Similar or identical products which may also be used are available under the trade names Nikkol® (e.g. Nikkol® HCO-40 and HCO-60), Mapeg® (e.g. Mapeg® CO-40h), Incrocas® (e.g. Incrocas® 40), Tagat® (for example polyoxyethylene-glycerol-fatty acid esters e.g. Tagat® RH 40; and Tagat® TO, a polyoxyethylene-glycerol-trioleate having a HLB value of 11.3; Tagat® RH 40 is preferred) and Simulsol OL-50 (PEG-40 castor oil, having a saponification value of about 55 to 65, an acid value of max. 2, an iodine value of 25 to 35, a water content of max. 8%, and an HLB of about 13, available from Seppic). These surfactants are further described in Fiedler loc. cit..
ii) Polyoxyethylene-sorbitan-fatty acid esters, for example mono- and tri-lauryl, palmityl, stearyl and oleyl esters of the type known and commercially available under the trade name Tween® (Fiedler, loc.cit. p.1615 ff) including the products Tween®
   20 [polyoxyethylene(20)sorbitanmonolaurate],
   21 [polyoxyethylene(4)sorbitanmonolaurate],
   40 [polyoxyethylene(20)sorbitanmonopalmitate],
   60 [polyoxyethylene(20)sorbitanmonostearate],
   65 [polyoxyethylene(20)sorbitantristearate],
   80 [polyoxyethylene(20)sorbitanmonooleate],
   81 [polyoxyethylene(5)sorbitanmonooleate],
   85 [polyoxyethylene(20)sorbitantrioleate].

   Especially preferred products of this class are Tween® 40 and Tween® 80.
iii) Polyoxyethylene fatty acid esters, for example polyoxyethylene stearic acid esters of the type known and commercially available under the trade name Myrj® (Fiedler, loc. cit., 2, p. 1042). An especially preferred product of this class is Myrj® 52 having a D²⁵ of about 1.1., a melting point of about 40 to 44°C, an HLB value of about 16.9., an acid value of about 0 to 1 and a saponification no. of about 25 to 35.
iv) Polyoxyethylene-polyoxypropylene co-polymers and block co-polymers, poloxamers, for example of the type known and commercially available under the trade names Pluronic®, Emkalyx® (Fiedler, loc. cit., 2, p. 1203). An especially preferred product of this class is Pluronic® F68 (poloxamer 188), having a melting point of about 52°C and a molecular weight of about 6800 to 8975. A further preferred product of this class is Synperonic® PE L44 (poloxamer 124).
v) Dioctylsodiumsulfosuccinate as known and commercially available under the trade mark Aerosol OT® from e.g. American Cyanamid Co. (Fiedler, loc. cit., 1, p. 118), or di-[2-ethylhexyl]-succinate (Fiedler, loc. cit., 1, p. 487).
vi) Phospholipids, in particular lecithins (Fiedler, loc. cit., 2, p. 910, 1184). Suitable lecithins include, in particular, soya bean lecithins.
vii) Sorbitan fatty acid esters, e.g. sorbitan mono C₁₂₋₁₈ fatty acid esters, or sorbitan tri C₁₂₋ ₁₈ fatty acid esters as known and commercially available under the trade mark Span® from e.g. ICI. An especially preferred product of this class is e.g. Span® 20 (sorbitan monolaurate) or Span® 80 (sorbitan monooleate) (Fiedler, loc. cit., 2, p. 1430; Handbook of Pharmaceutical Excipients, loc. cit., page 473).
viii) Polyoxyethylene mono esters of a saturated C₁₀ to C₂₂, e.g. C₁₈ substituted e.g. hydroxy fatty acid; e.g. 12 hydroxy stearic acid PEG ester, e.g. of PEG about e.g. 600-900 e.g. 660 daltons MW, e.g. Solutol® HS 15 from BASF, Ludwigshafen, Germany.
ix) Polyoxyethylene alkyl ethers, e.g. polyoxyethylene glycol ethers of C₁₂ to C₁₈ alcohols, e.g. Polyoxyl 2-, 10- or 20-cetyl ether or Polyoxyl 4- or 23-lauryl ether, or polyoxyl 2-, 10- or 20-oleyl ether, or Polyoxyl 2-, 10-, 20- or 100-stearyl ether, as known and commercially available under the trade mark Brij® from e.g. ICI. An especially preferred product of this class is e.g. Brij® 35 (Polyoxyl 23 lauryl ether) or Brij® 98 (Polyoxyl 20 oleyl ether) (Fiedler, loc. cit., 1, pp. 259; Handbook of Pharmaceutical Excipients, loc. cit., page 367).
   Similar products which may also be used are polyoxyethylene-polyoxypropylene-alkyl ethers, e.g. polyoxyethylene-polyoxypropylene- ethers of C₁₂ to C₁₈ alcohols, e.g. polyoxyethylen-20-polyoxypropylene-4-cetylether which is known and commercially available under the trade mark Nikkol PBC® 34, from e.g. Nikko Chemicals Co., Ltd. (Fiedler, loc. cit., vol. 2, pp. 1239).
x) Water soluble tocopheryl polyethylene glycol succinic acid esters (TPGS), e.g. with a polymerisation number ca 1000, e.g. available from Eastman Fine Chemicals Kingsport, Texas, USA.
xi) Polyglycerol fatty acid esters, with e.g. from 2 to 20, e.g. 10 glycerol units. The fatty acid constituent may include both saturated and unsaturated fatty acids having a chain length of from e.g. C₈-C₁₈. Particularly suitable is e.g. decaglycerylmonolaurat or decaglycerylmonomyristat, as known and commercially available under the trade mark Decaglyn® 1-L or Decaglyn® I-M, respectively, from e.g. Nikko Chemicals C., Ltd (Fiedler, loc. cit., vol. 2, pp. 1228).
xii) Polyethylene glycol glyceryl fatty acid ester. The fatty acid ester may include mono and/or di and/or tri fatty acid ester. The fatty acid constituent may include both saturated and unsaturated fatty acids having a chain length of from e.g. C₁₂-C₁₈. The polyethylene glycols may have e.g. from 10 to 40 [CH₂-CH₂-O] units, e.g. 15 or 30 units. Particularly suitable is polyethylene glycol (15) glyceryl monostearat or polyethylene glycol (15) glyceryl monooleate which is commercially available, e.g. under the trade name TGMS®-15 or TGMO®-15, respectively, e.g. from Nikko Chemicals Co., Ltd. Further suitable is polyethylene glycol (30) glyceryl monooleate which is commercially available, e.g. under the trade name Tagat® O, e.g. from Goldschmidt (H. Fiedler, loc cit, vol. 2, p. 1502-1503).
xiii) Sterols and derivatives thereof, for example cholesterols and derivatives thereof, in particular phytosterols, e.g. products comprising sitosterol, campesterol or stigmasterol, and ethylene oxide adducts thereof, for example soya sterols and derivatives thereof, e.g. polyethylene glycol sterols, e.g. polyethylene glycol phytosterols or polyethylene glycol soya sterols. The polyethylene glycols may have e.g. from 10 to 40 [CH₂-CH₂-O] units, e.g. 25 or 30 units. Particularly suitable is polyethylene glycol (30) phytosterol which is commercially available, e.g. under the trade name Nikkol BPS®-30, e.g. from Nikko Chemicals Co., Ltd. Further suitable is polyethylene glycol (25) soya sterol which is commercially available, e.g. under the trade name Generol® 122 E 25, e.g. from Henkel (H. Fiedler, loc cit, vol. 1, p. 680).

It is to be appreciated that surfactants may be complex mixtures containing side products or unreacted starting products involved in the preparation thereof, e.g. surfactants made by polyoxyethylation may contain another side product, e.g. polyethylene glycol.

A surfactant having a hydrophilic-lipophilic balance (HLB) value of 8 to 17 is preferred. The HLB value is preferably the mean HLB value.

The surfactant selected preferably has a hydrophilic-lipophilic balance (HLB) of at least 10, for example Cremophor.

In one aspect the present invention provides a composition according to the present invention wherein the surfactant is selected from the group consisting of (i) reaction products of natural or hydrogenated vegetable oil and ethylene oxide, and (ii) polyoxyethylene sorbitan fatty acid esters.

Preferably the relative proportion of the second component(s), the lipophilic component and the surfactant lie within the "microemulsion" region on a standard three way plot. The compositions thus obtained are microemulsion preconcentrates of high stability that are capable, on addition to water, of providing microemulsions having a mean particle size of < 200 nm.

Standard three way plots, e.g. phase diagrams, can be generated in a conventional manner as described in e.g. GB patent publication no. 2 222 770 or WO 96/13273.

In a further alternative aspect, the present invention relates to a cyclosporin or macrolide preparation, which comprises
1) a cyclosporin or macrolide as an active ingredient;
2) a second component as specified above;
3) one or a mixture of two or more selected from the group consisting of an esterified compound of fatty acid and primary alcohol, medium chain fatty acid triglyceride and fatty acid monoglyceride as an oil component, and
4) a surfactant having an HLB (Hydrophilic Lipophilic Balance) value of 8 to 17, e.g. in a gelatin shell containing polyethylene glycol and propylene glycol as a plasticizer.

In another aspect the present invention provides a cyclosporin or macrolide preparation, which comprises a composition containing
1) cyclosporin or macrolide as an active ingredient, and 2) the second component as decribed above.

Although any pharmaceutically acceptable components selected from the group specified above as secondary components may be used in such a composition, certain components are preferred. These include Sunfat®GDC-N, Lauroglycol®90, oleyl alcohol.

Accordingly, in another aspect the present invention provides a cyclosporin or macrolide preparation, which comprises a composition containing
1) cyclosporin or macrolide as an active ingredient, and 2) a second component selected from the group consisting of
   (i) glyceryl di C₆-C₁₆ fatty acid ester,
   (ii) propylene glycol mono C₆-C₁₂ fatty acid ester,
   (iii) fatty alcohols.

Such a composition, which is also a composition of the invention, may optionally additionally comprise any other component as described herein, if desired in the amounts described herein.

The compositions may be formulated in conventional manner using recipies as disclosed in WO 97/36610 (PCT/KR/98) replacing the propylene carbonate by the secondary component.

If desired, polyethylene glycol which has a high boiling point, is non-volatile, and a solvent for cyclosporin, can also be present. In the composition according to the present invention, although any polyethylene glycol which can be liquified can be used, polyethylene glycol (PEG) having molecular weight of 200 to 600, particularly PEG 200, can be preferably used.

In the present invention, in a further alternative aspect a mixture of polyethylene glycol and the second component may be used as the component in the present invention, and can be generally combined in the ratio of 1:0.1-5, preferably 1:0.1-3, most preferably 1:0.2-2, on the basis of weight.

In the composition of the present invention, in a further alternative aspect the second component may be used preferably in the ratio of 0. 1 to 10 parts by weight, more preferably 0.5 to 8 parts by weight, and most preferably 1 to 5 parts by weight, per 1 part by weight of cyclosporin.

The third component which may be used in the emulsion, e.g. microemulsion, pre-concentrate according to the present invention is a lipophilic, e.g. oil, component. As the lipophilic, e.g. oil, component in the present invention, one or a mixture of two or more selected from the group consisting of esterified compounds of fatty acid and primary alcohol, medium chain fatty acid triglycerides (when present) and fatty acid monoglycerides can be used. The esterified compound of fatty acid and primary alcohol which can be used in the present invention may include an esterified compound of fatty acid having 8 to 20 carbon atoms and primary alcohol having 2 to 3 carbon atoms, for example, isopropyl myristate, isopropyl palmitate, ethyl linoleate, ethyl oleate, etc., with an esterified compound of linoleic acid and ethanol being particularly preferable. In addition, as the medium chain fatty acid triglyceride (when present) a triglyceride of saturated fatty acid having 8 to 10 carbon atoms can be used with caprylic/capric acid triglyceride as a vegetable oil triglyceride of saturated fatty acid being most preferably used. The fatty acid monoglyceride which can also be used as the lipophilic, e.g. oil, component in the present invention includes a monoglyceride of fatty acid having 18 to 20 carbon atoms, particularly monoglyceride of oleic acid.

In a microemulsion pre-concentrate according to the present invention, the lipophilic, e.g. oil, component may be used in the ratio of 1 to 10 parts by weight, preferably 2 to 6 parts by weight, per 1 part by weight of cyclosporin.

In a further alternative aspect preferably fatty acid monoglyceride and fatty acid ester are present as lipophilic, e.g. oil, component, e.g. in the ratio 1 : 1 to 1 : 2, e.g. 1 : 1 to 1 : 1.2.

In a further alternative aspect optionally caprylic/capric acid triglyceride is also present e.g. in a ratio to ethyl linoleate of from 1 : 0.05 to 1, e.g. 1 : 0.1 to 0.2.

In the oil mixture used as the lipophilic, e.g. oil, component according to the present invention, the mixing ratio of fatty acid monoglyceride: an esterified compound of fatty acid and primary alcohol: medium chain fatty acid triglyceride (when present) may be generally in the range of 1 : 0.1-5 : 0.1-10 preferably in the range of 1 : 0.1-3.0 : 0.1-3.0, on the basis of weight.

The fourth component which may be used in the composition according to the present invention is a surfactant. The suitable surfactants for use in the present invention include any of pharmaceutically acceptable surfactants having an HLB (Hydrophilic Lipophilic Balance) value of 8 to 17, which are capable of stably emulsifying the lipophilic portion of the composition comprising the cyclosporin-containing lipophilic, e.g. oil, component and the portion comprising the second component and the co-surfactant in water to form a stable microemulsion. Examples of the preferred surfactant according to the present invention include polyoxyethylene products of natural or hydrogenated vegetable oils, polyoxyethylene-sorbitan-fatty acid esters, and the like, for example, Nikkol® HCO-50, Nikkol® HCO-40, Nikkol® HCO-60, Tween® 20, Tween® 21, Tween® 40, Tween® 60, Tween® 80, Tween® 81, etc. For example, a polyoxyethylene (50)- or polyoxyethylene (40)- hydrogenated castor oil which is commercialised under the trade mark Nikkol® HCO-50 or Nikkol® HCO-40, respectively, (NIKKO Chemical Co., Ltd.) and a polyoxyethlyene (20) sorbitan monolaurate which is commercialised under the trade mark Tween® 20 (ICI Chemicals), having an acid value below 1, a saponification value of about 48-56, a hydroxyl value of about 45-55 and pH value (5%) of 4.5-7.0, can be preferably used.

In a further alternative aspect the surfactant can include any one of the above-mentioned surfactants alone or, preferably, in a combination of two or more surfactants selected from the above surfactants. In the composition according to the present invention, the surfactants can be used in the ratio of 1 to 10 parts by weight, preferably in the ratio of 2 to 8 parts by weight, per 1 part by weight of cyclosporin.

In addition, when the mixture of two surfactants, i.e. polyoxyethylene (50) hydrogenated castor oil and polyoxyethylene (20) sorbitan monolaurate is used in the composition of the present invention, the constitutional ratio of polyoxyethylene (50) hydrogenated castor oil : polyoxyethylene (20) sorbitan monolaurate is preferably in the range of 1:0.1-5, more preferably in the range of 1:0.5-4, most preferably in the range of 1:0.1-0.25, on the basis of weight.

In a further alternative aspect in the composition according to the present invention, up to four components are present preferably in the ratio of cyclosporin or macrolide: second component: lipophilic, e.g. oil, component: surfactant = 1 : 0.1- 10 : 1-10 : 1-10, and more preferably in the ratio of cyclosporin or macrolide: second component: lipophilic, e.g. oil, component : surfactant = 1 : 0.5-8: 2-6 : 2-8, by weight.

Another substance which can e.g. be used present is a hydrophilic cosurfactant e.g. a polyoxyethylene-polyoxypropylene block copolymer, poloxamer, which is liquid at room temperature. Such block copolymer has been commercialized under the trade name Pluronic® L10, L31, L35, L43, L44 (poloxamer 124), L101, 31R1, with poloxamer 124 which is pharmaceutically acceptable being preferably used as the mixture with the secondary component specified above. Poloxamer 124 is also available under trade name Lutrol® or Synperonic® PE L44. Poloxamer is a hydrophilic, high molecular surfactant having molecular weight of 2000 to 18000, which may be used as a solvent for medicinal components, lipid emulsions, ointment base, binders or coating agents for tablets, gelling agent, etc. Although the properties of poloxamers depend on their series, they are thermally stable with a flash point of 260°C. Poloxamer 124 dissolves easily in organic solvents such as propylene glycol or xylene, in contrast to other poloxamers. In addition, in comparison with all other solvents used in prior art cyclosporin formulations, poloxamer is not hygroscopic. It thus does not change the constitutional ratio by dissolution of gelatin shell, permeation or evaporation.

In one aspect the present invention provides a composition according to the present invention wherein a hydrophilic cosurfactant, e.g. a polyoxyethylene polyoxypropylene block copolymer is additionally present.

In a further alternative aspect when a mixture of the second component and polyoxyethylene-polyoxypropylene block copolymer in a liquid state at room temperature is used in the second component in the present invention, they may be combined in the ratio of 1:0.1-5, preferably 1:0.1-3, more preferably 1:0.1-1, on the basis of weight. Typically it is present in a concentration of from 5 to 10%.

In one aspect the present invention provides a composition according to the present invention wherein the second component and the hydrophilic cosurfactant, e.g. a polyoxyethylene polyoxypropylene block copolymer, are combined in the ratio of 1 : 0.1 to 5 on the basis of weight.

In the pharmaceutical composition according to the present invention, in a further alternative aspect the ratio of cyclosporin to the hydrophilic cosurfactant is preferably in the range of 1:0.1-1, more preferably 1:0.5-0.8, on the basis of weight.

The emulsion, e.g. microemulsion, preconcentrate compositions, e.g. those in the examples hereinafter, may show good stability characteristics as indicated by standard stability trials, for example having a shelf life stability of up to one, two or three years, and even longer. The microemulsion preconcentrate compositions of this invention produce stable microemulsions, e.g, for up to one day or longer, e.g. one day.

The pharmaceutical composition may also include further additives or ingredients, for example antioxidants (such as ascorbyl palmitate, butyl hydroxy anisole (BHA), butyl hydroxy toluene (BHT) and tocopherols) and/or preserving agents. In a further alternative aspect these additives or ingredients may comprise about 0.05 to 1 % by weight of the total weight of the composition. The pharmaceutical composition may also include sweetening or flavoring agents in an amount of up to about 2.5 or 5% by weight based on the total weight of the composition. Preferably the antioxidant is α-tocopherol (vitamin E).

Details of excipients of the invention are described in Fiedler, H. P., loc cit: "Handbook of Pharmaceutical Excipients", loc cit; or may be obtained from the relevant manufacturers.

Any carbon chain not otherwise specified herein conveniently contains 1 to 18 carbon atoms, e.g. 10 to 18 carbon atoms, when a terminal group or 2 or 3 carbon atoms when a polymer moiety.

It will be appreciated that the present invention encompasses
a) in respect of component 2) any of components i) to v) individually or in combination with one, two or more of the other components i) to v),
b) in respect of component 3) any of the lipophilic components specified above individually or in combination,
c) in respect of component 4) any of the surfactants specified above, e.g. surfactants i) to xiii), individually or in combination.

The pharmaceutical compositions exhibit especially advantageous properties when administered orally; for example in terms of consistency and high level of bioavailability obtained in standard bioavailability trials, e.g. 2 to 4 times higher than emulsions. These trials are performed in animals e.g. rats or dogs or healthy volunteers using HPLC or a specific or nonspecific monoclonal kit to determine the level of the drug substance, e.g. macrolide in the blood. For example, the composition of Example 1 administered p.o. to dogs may give surprisingly high Cₘₐₓ values as detected by ELISA using a specific monoclonal antibody.

In one aspect the present invention provides a composition according to the present invention for use in medicine.

Pharmacokinetic parameters, for example absorption and blood levels, also become surprisingly more predictable and problems in administration with erratic absorption may be eliminated or reduced. Additionally the pharmaceutical compositions are effective with tenside materials, for example bile salts, being present in the gastro-intestinal tract. That is. the pharmaceutical compositions are fully dispersible in aqueous systems comprising such natural tensides and thus capable of providing micro emulsion systems in situ which are stable and do not exhibit precipitation of the active agent or other disruption of fine particulate structure. The function of the pharmaceutical compositions upon oral administration remain substantially independent of and/or unimpaired by the relative presence or absence of bile salts at any particular time or for any given individual.

The compositions of this invention reduce variability in inter- and intra-patient dose response.

In a further alternative aspect the invention also provides a process for the production of a pharmaceutical composition as defined above, which process comprises bringing (1) the second component; (2) the lipophilic component; and (3) the surfactant into intimate admixture, and adding the active agent, e.g. cyclosporin or the compound of the macrolide class. When required, the composition may be compounded into unit dosage form, for example filling the composition into gelatine capsules.

Optionally further components or additives, in particular a hydrophilic component co-component, for example ethanol, may be mixed with components (1), (2) and (3) or with or after addition of active agent.

The composition may be combined with water or an aqueous solvent medium such that an emulsion, e.g. microemulsion, is obtained.

The present applicants also contemplate emulsion, e.g. microemulsion, preconcentrate compositions which may be free of refined fish oil and/or ethanol and/or transesterified ethoxylated vegetable oil.

It has also been found that stable compositions containing macrolides may be obtained by formulating the macrolide in an acidic environment. Compositions are understood herein to be stable when the macrolide drug substance remains substantially intact after a period of days or weeks at room temperature (25°C).

The acid may be lipid soluble and/or ethanol soluble. The acid may be for example a fatty acid, e.g. oleic acid. The acid may be a carboxylic acid, for example a mono-, di- or tricarboxylic acid, and preferably a mono- or dicarboxylic acid. The acid may comprise one or more hydrophilic groups, e.g. hydroxy groups, and preferably one or two hydrophilic groups. Suitable acids for use in this invention include malonic acid, fumaric acid, maleic acid, D-malic acid, L-malic acid, citric acid, ascorbic acid, succinic acid, oxalic acid, benzoic acid or lactic acid or an acid with a similar pKa, e.g. 2-7. Preferred acids include malonic acid, oxalic acid, citric acid and lactic acid. Malonic acid is more preferred.

The preferred amount of acid may be determined by routine experimentation. The ratio by weight of macrolide to acid in the compositions of this invention may be up to 20:1, for example from 1:5 to 5:1, e.g. 1:1. In a further alternative aspect the acid may be present in an amount of between 0.05% and 5% by weight of the composition.

In a further alternative aspect the macrolide may be present in an amount of 1 to 15 % by weight of the composition.

The type of pharmaceutical composition is not critical. It may be solid, but it is preferably liquid. The macrolide may, for example, be formulated into an emulsion, e.g. microemulsion, preconcentrate or emulsion preconcentrate as defined above, and combined with an amount of acid. The acid-stabilised composition may be administered enterally, e.g orally, e.g. as a capsule or drink solution, or parenterally, e.g. as an infusion concentrate. Oral administration is preferred.

The utility of all the pharmaceutical compositions of the present invention may be observed in standard clinical tests in, for example, known indications of active agent dosages giving equivalent blood levels of active agent; for example using dosages in the range of 2.5 mg to 1000 mg of active agent per day for a 75 kilogram mammal, e.g. adult and in standard animal models. The increased bioavailability of the active agent provided by the compositions may be observed in standard animal tests and in clinical trials, e.g. as described above.

The optimal dosage of active agent to be administered to a particular patient must be considered carefully as individual response to and metabolism of the macrolide compound, c.g. rapamycin, may vary. It may be advisable to monitor the blood serum levels of the active agent by radioimmunoassay, monoclonal antibody assay, or other appropriate conventional means. Dosages of a macrolide will generally range from 1 to 1000 mg per day, e.g. 2.5 mg to 1000 mg per day for a 75 kilogram adult, preferably 25 mg to 500 mg, with the optimal dosage being approximately 50 to 100 mg per day. Satisfactory results are obtained by administering about 75 mg per day for example in the form of two capsules, one containing 50 mg and one containing 25 mg; or three capsules each containing 25 mg. Cyclosporin dosages may be 25 to 1000 mg per day (preferably 50 mg to 500 mg) and the FK 506 dosage may be 2.5 mg to 1000 mg per day (preferably 10 mg to 250 mg). A daily dosage of between 0.5 and 5 mg/kg body weight/day is indicated for administration of 40-0-(2-hydroxy)ethyl rapamycin.

The pharmaceutical compositions are preferably compounded in unit dosage form, for example by filling them into orally administrable capsule shells. The capsule shells may be soft or hard gelatine capsule shells. Where the pharmaceutical composition is in unit dosage form, each unit dosage will suitably contain between 10 and 100 mg of the active agent, more preferably between 10 and 50 mg; for example 15, 20, 25, or 50 mg. Such unit dosage forms are suitable for administration 1 to 5 times daily depending upon the particular purpose of therapy, the phase of therapy and the like.

However, if desired, the pharmaceutical compositions may be in drink solution form and may include water or any other aqueous system, to provide emulsion, e.g. microemulsion, systems suitable for drinking.

The pharmaceutical compositions are particularly useful for treatment and prevention of the conditions disclosed at pages 40 and 41 in EP 427 680, and at pages 5 and 6 in PCT/EP93/02604.

The pharmaceutical compositions are particularly useful for:
a) treatment and prevention of organ or tissue transplant rejection, for example for the treatment of the recipients of heart, lung, combined heart-lung, liver, kidney, pancreatic, skin or corneal transplants. The pharmaceutical compositions are also indicated for the prevention of graft-versus-host disease, such as sometimes occurs following bone marrow transplantation;
b) treatment and prevention of autoimmune disease and of inflammatory conditions, in particular inflammatory conditions with an aetiology including an autoimmune component such as arthritis (for example rheumatoid arthritis, arthritis chronic progrediente and arthritis deformans) and rheumatic diseases; and
c) treatment of multi-drug resistance (MDR).

In a further aspect the present invention provides the use of a composition according to the present invention in the manufacture of a medicament for the treatment and prevention of an autoimmune or inflammatory condition or for the treatment and prevention of transplant rejection or for the treatment of multi-drug resistance.

The macrolide active agents also exhibit anti-tumour and antifungal activity and hence the pharmaceutical compositions can be used as anti-tumour and anti-fungal agents.

Each of the exemplified compounds may be used as a macrolide in the examples listed below.

### Examples

Following is a description by way of example of compositions of this invention. Unless otherwise indicated, components are shown in % by weight based on each composition.
Miglyol® 812 is from the Condea company, Germany.
Labrafil® M 2125 CS, Gelucire® 44/14, Maisine®, Lauroglycol® 90 are from the Gattefossé company, France.
Tegin® O is a representative mono-glyceride which is ca 55-65 % pure, the remainder containing diglycerides, obtainable from Goldschmitt, Essen, Germany.
Neobee® M-5 F is from Stepan Europe, France.
GMOrphic® 80 is a representative mono-glyceride which is ca. 94% pure, the remainder containing diglycerides, obtainable from Eastman Chemicals Co., Kingsport, CN, USA.
Synperonic® PE L44 is a representative poloxamer from ICI, UK.
Simulsol® OI-50 is from Seppic, France.
Cremophor® RH 40 is from BASF, Germany.
Sunfat® GDC-N is from Taiyo Kagaku Co., Japan.
Cetiol® HE is from Henkel KGaA, Germany.

Particle size measurements are made at 20°C at a dilution of 1 ml composition in 10 to 100 ml water by photon correlation spectroscopy using, for example a Malvern ZetaSizer No. 3 from Malvern Instruments.

### Example 1:

### Preparation of oral drink solutions

Compositions are made up with the following components. Some compositions are turbid but, optionally on warming to 40°C, on dilution of 1:10 give a non-opaque, or translucent liquid, which contains particles the size of which are measured by a Zetasizer.

Compositions I-VI, XIII-XX are not within the scope of the claims.

| COMPONENT | QUANTITY | | | |
|---|---|---|---|---|
| | I % | II % | III % | IV % |
| Cyclosporin A | 10 | 10 | 10 | 10 |
| Triethylcitrate | 10 | 14 | 5 | 5 |
| Cremophor® RH40 | 40 | 43.75 | 43.11 | 42.5 |
| Synperonic®PE L44 | 5.71 | 6.00 | 6.07 | 6.07 |
| GMOrphic® 80 | 8 | 2 | - | 8.5 |
| Tegin® O | - | - | 8.5 | - |
| Labrafil® M 2125 CS | 5.71 | 6.25 | 5.10 | 6.07 |
| Miglyol® 812 | 20.57 | 18.0 | 21.86 | 21.86 |
| Alpha-tocopherol | - | - | 0.36 | - |
| mean Droplet size (nm) | 30 | 36 | 32 | 29 |

| | V % | VI % | | |
|---|---|---|---|---|
| Cyclosporin A | 10 | 10 | | |
| N-methylpyrrolidone | 9 | 9 | | |
| Simulsol® OL-50 | 63 | 54 | | |
| Neobee® M-5 F | 18 | 27 | | |
| mean Droplet size (nm) | 28 | 37 | | |

| | VII % | VIII % | IX % | X % |
|---|---|---|---|---|
| Cyclosporin A | 10 | 5 | 10 | 8 |
| Cremophor® RH40 | 45 | 22.5 | 45 | 36 |
| Sunfat® GDC-N | 27 | 13.5 | - | - |
| Lauroglycol® 90 | - | - | 36 | 28.8 |
| Propylene glycol | 18 | 9 | 9 | 7.2 |
| Gelucire® 44/14 | - | 50 | - | 20 |
| mean Droplet size (nm) | 33 | 36 | 31 | 21 |

| | XI % | XII % | XIII % | XIV % |
|---|---|---|---|---|
| Cyclosporin A | 10 | 8 | 10 | 8 |
| Cremophor® RH40 | 54 | 43.2 | 63 | 50.4 |
| Oleyl alcohol | 18 | 14.4 | - | - |
| Oleic acid | - | - | 18 | 14.4 |
| Propylene glycol | 18 | 14.4 | 9 | 7.2 |
| Gelucire® 44/14 | - | 20 | - | 20 |
| mean Droplet size (nm) | 26 | 18 | 39 | 18 |

| | XV % | XVI % | XVII % | XVIII % |
|---|---|---|---|---|
| Cyclosporin A | 10 | 10 | 10 | 10 |
| Cremophor® RH40 | 38.6 | 38.6 | 38.6 | 38.6 |
| Cetiol® HE | 9.5 | - | - | - |
| Acetyl triethyl citrate | - | 9.5 | - | - |
| N-Methylpyrrolidone | - | - | 9.5 | - |
| Benzyl alcohol | - | - | - | 9.5 |
| Ethanol | 9.5 | 9.5 | 9.5 | 9.5 |
| Maisine® | 32.4 | 32.4 | 32.4 | 32.4 |
| mean Droplet size (nm) | 34 | 25 | 31 | 30 |

| | XIX % | XX % | | |
|---|---|---|---|---|
| Cyclosporin A | 10 | 10 | | |
| Cremophor® RH40 | 38.6 | 38.6 | | |
| Propylene glycol | 9.5 | 9.5 | | |
| Ethanol | 9.5 | 9.5 | | |
| Oleic acid | 17.4 | 17.4 | | |
| Labrafil® 1944CS | 15 | - | | |
| Labrafil® 2125 CS | - | 15 | | |
| mean Droplet size (nm) | 29 | 31 | | |

These compositions may be encapsulated in hard and soft gelatine capsules.

Other examples may be made excluding Synperonic® PE L44, GMOrphic® 80, Tegin® O, Labrafil® M 2125 CS, Alpha-tocopherol, ethanol, propylene glycol.
Further examples may be made replacing triethyl citrate, N-Methylpyrrolidone, Sunfat® GDC-N, Lauroglycol® 90, oleyl alcohol, Cetiol® HE, acetyl triethyl citrate, benzyl alcohol, or oleic acid by any of the second components specified above.

The examples illustrate compositions useful for example in the prevention of transplant rejection or for the treatment of autoimmune disease, on administration of from 1 to 5 unit dosages/day at a dose of 2 to 5 mg/kg per day. The examples are described with particular reference to Ciclosporin but equivalent compositions may be obtained employing any macrolide or other active agent.

On visual inspection after dilution, each of the compositions forms a clear and stable microemulsion.

## Claims

1. A composition in form of an emulsion pre-concentrate or a microemulsion pre-concentrate suitable for oral administration comprising:
1) a cyclosporine or macrolide, and a carrier medium comprising
2) a second component selected from the group consisting of
(i) glyceryl di C₆-C₁₆ fatty acid ester,
(ii) glyceryl mono C₆-C₁₄ fatty acid ester,
(iii) a mixture of mono-, diglycerides of C₆-C₁₆ fatty acids,
(iv) propylene glycol mono C₆-C₁₂ fatty acid ester,
(v) fatty alcohols, and
3) a lipophilic component, and
4) a surfactant,
with the proviso that when component 2) consists of a mixture of mono-, diglycerides of C₈-C₁₀ fatty acids, said composition is free of a C₆-C₁₂ fatty acid triglyceride.

2. A composition according to claim 1, in the form of a microemulsion pre-concentrate.

3. A composition according to claim 1 or claim 2, wherein the second component comprises a polyethylene glycol glyceryl C₆-C₁₀ fatty acid ester having from 5 to 10 [CH₂-CH₂-O] units.

4. A composition according to any preceding claim wherein the lipophilic component is selected from the group consisting of
(i) transesterified ethoxylated vegetable oil,
(ii) mixed mono-, di- and tri-glycerides,
(iii) propylene glycol mono- and di-fatty acid esters, and
(iv) esterified compounds of fatty acid and primary alcohol.

5. A composition according to any preceding claim further comprising one or more antioxidants selected from the group consisting of ascorbyl palmitate, butyl hydroxy anisole (BHA), butyl hydroxy toluene (BHT) and tocopherols in an amount of 0.05 to 1 % by weight of the total weight of the composition.

6. A composition according to claim 5 wherein the antioxidant present is α-tocopherol.

7. A composition according to any one of the preceding claims comprising the cyclosporine or macrolide in an amount of 1 to 15% by weight of the composition.

8. A composition according to any one of the preceding claims comprising the second component in an amount of 5 to 50 %, the lipophilic component in an amount of 5 to 85 %, and the surfactant in an amount of 5 to 80 % by weight of the carrier medium.

9. A composition according to any preceding claim, the relative proportion of the cyclosporine or macrolide, the second component, the lipophilic component and the surfactant in said composition being such that upon dilution with water to a ratio of 1 part by weight of said composition to 1 to 10 parts by weight of water, and oil-in-water microemulsion having particles of a mean size of less that 200 nm, is spontaneously formed.

10. A composition according to any preceding claim wherein the cyclosporine is Cyclosporin A.

11. A composition according to any preceding claim wherein the surfactant is selected from the group consisting of (i) reaction products of natural or hydrogenated vegetable oil and ethylene oxide, and (ii) polyoxyethylene sorbitan fatty acid esters.

12. A composition according to any preceding claim wherein the ratio of the cyclosporine or macrolide: second component : lipophilic component : surfactant is 1 : 0.1 to 10 : 1 to 10 :1 to 10 on the basis of weight.

13. A composition according to any preceding claim wherein a hydrophilic cosurfactant is additionally present.

14. A composition according to claim 13 wherein the hydrophilic cosurfactant is polyoxyethylene polyoxypropylene block copolymer.

15. A composition according to claim 13 or 14 wherein the second component and the hydrophilic cosurfactant are combined in the ratio of 1 : 0.1 to 5 on the basis of weight.

16. A composition of any preceding claim in unit dosage form.

17. A composition according to any preceding claim for use in medicine.

18. Use of a composition according to any preceding claim for the manufacture of a medicament for the treatment or prevention of tissue or organ transplant rejection, or graft-versus-host disease.

19. Use of a composition according to any one of claims 1 to 17 for the manufacture of a medicament for the treatment or prevention of an autoimmune or inflammatory condition.

20. A soft or hard gelatin capsule comprising a composition as defined in any of claims 1 to 17.

## Patentansprüche

1. Zusammensetzung in Form eines Emulsionsvorkonzentrats oder eines Mikroemulsionsvorkonzentrats zur oralen Verabreichung, umfassend
1) ein Cyclosporin oder Makrolid und ein Trägermedium, umfassend
2) eine zweite Komponente, welche ausgewählt ist aus der Gruppe, die besteht aus
(i) Glyceryldi-C₆-C₁₆-fettsäureester,
(ii) Glycerylmono-C₆-C₁₄-fettsäureester,
(iii) einem Gemisch aus Mono- und Diglyceriden von C₆-C₁₆-Fettsäuren,
(iv) Propylenglycolmono-C₆-C₁₂-fettsäureester und
(v) Fettalkoholen,
3) eine lipophile Komponente und
4) ein Tensid,
mit der Maßgabe, dass diese Zusammensetzung frei ist an einem C₆-C₁₂-Fettsäuretriglycerid, falls die Komponente 2) aus einem Gemisch aus Mono- und Diglyceriden von C₈-C₁₀-Fettsäuren besteht.

2. Zusammensetzung nach Anspruch 1 in Form eines Mikroemulsionsvorkonzentrats.

3. Zusammensetzung nach Anspruch 1 oder 2, worin die zweite Komponente einen Polyethylenglycolglyceryl-C₆-C₁₀-fettsäureester umfasst, der 5 bis 10 [CH₂-CH₂-O]-Einheiten aufweist.

4. Zusammensetzung nach einem der vorhergehenden Ansprüche, worin die lipophile Komponente ausgewählt ist aus der Gruppe, die besteht aus
(i) umgeestertem ethoxyliertem Pflanzenöl,
(ii) einem Gemisch aus Mono-, Di- und Triglyceriden,
(iii) Propylenglycolmono- und -difettsäureestern und
(iv) veresterten Verbindungen von Fettsäure und primärem Alkohol.

5. Zusammensetzung nach einem der vorhergehenden Ansprüche, die ferner umfasst ein oder mehr Antioxidantien, welche aus der Gruppe ausgewählt sind, die besteht aus Ascorbylpalmitat, Butylhydroxyanisol (BHA), Butylhydroxytoluol (BHT) und Tocopherolen, in einer Menge von 0,05 bis 1 Gew.-% des Gesamtsgewichts der Zusammensetzung.

6. Zusammensetzung nach Anspruch 5, worin das vorhandene Antioxidans α-Tocopherol ist.

7. Zusammensetzung nach einem der vorhergehenden Ansprüche, welche das Cyclosporin oder Makrolid in einer Menge von 1 bis 15 Gew.-% der Zusammensetzung umfasst.

8. Zusammensetzung nach Anspruch 1 oder 2, welche die zweite Komponente in einer Menge von 5 bis 50 Gew.-%, die lipophile Menge in einer Menge von 5 bis 85 Gew.-% und das Tensid in einer Menge von 5 bis 80 Gew.-% des Trägermediums umfasst.

9. Zusammensetzung nach einem der vorhergehenden Ansprüche, wobei der relative Anteil des Cyclosporins oder Makrolids, der zweiten Komponente, der lipophilen Komponente und des Tensids in der Zusammensetzung derart ist, dass nach Verdünnung mit Wasser auf ein Verhältnis von 1 Gew.-Teil der Zusammensetzung zu 1 bis 10 Gew.-Teilen Wasser spontan eine Öl-in-Wasser Mikroemulsion mit Teilchen einer mittleren Größe von weniger als 200 nm gebildet wird.

10. Zusammensetzung nach einem der vorhergehenden Ansprüche, worin das Cyclosporin Cyclosporin A ist.

11. Zusammensetzung nach einem der vorhergehenden Ansprüche, worin das Tensid aus der Gruppe ausgewählt ist, die besteht aus (i) Reaktionsprodukten aus natürlichem oder hydriertem Pflanzenöl und Ethylenoxid und (ii) Polyoxyethylensorbitanfettsäureestern.

12. Zusammensetzung nach einem der vorhergehenden Ansprüche, worin das Verhältnis von Cyclosporin oder Makrolid : zweiter Komponente : lipophiler Komponente: Tensid 1 : 0,1 bis 10 : 1 bis 10:1 bis 10 auf Gewichtsbasis beträgt.

13. Zusammensetzung nach einem der vorhergehenden Ansprüche, worin ferner ein hydrophiles Cotensid vorhanden ist.

14. Zusammensetzung nach Anspruch 13, worin das hydrophile Cotensid ein Polyoxyethylen-Polyoxypropylen-Blockcopolymer ist.

15. Zusammensetzung nach Anspruch 13 oder 14, worin die zweite Komponente und das hydrophile Cotensid in einem Verhältnis von 1 : 0,1 bis 5 auf Gewichtsbasis kombiniert sind.

16. Zusammensetzung nach einem der vorhergehenden Ansprüche in Einheitsdosierungsform.

17. Zusammensetzung nach einem der vorhergehenden Ansprüche zur Verwendung in der Medizin.

18. Verwendung einer Zusammensetzung nach einem der vorhergehenden Ansprüche bei der Herstellung eines Arzneimittels zur Behandlung oder Verhinderung einer Gewebe- oder Organtransplantatabstoßung oder einer Transplantat-gegen-Wirt Erkrankung.

19. Verwendung einer Zusammensetzung nach einem der Ansprüche 1 bis 17 bei der Herstellung eines Arzneimittels zur Behandlung oder Verhinderung eines autoimmunen oder inflammatorischen Zustands.

20. Weichgelatinekapsel oder Hartgelatinekapsel, umfassend eine Zusammensetzung gemäß Definition nach einem der Ansprüche 1 bis 17.

## Revendications

1. Composition sous forme d'un préconcentré d'émulsion ou d'un préconcentré de microémulsion pour administration orale comprenant :
1) une cyclosporine ou un macrolide, et un milieu véhicule comprenant
2) un second composant sélectionné à partir du groupe comprenant
(i) un di-ester d'acide gras en C₆-C₁₆ et de glycérol,
(ii) un mono-ester d'acide gras en C₆-C₁₄ et de glycérol,
(iii) un mélange de mono-, di-glycérides d'acides gras en C₆₋C₁₆,
(iv) un mono-ester d'acide gras en C₆-C₁₂ et de propylèneglycol,
(v) des alcools gras, et
3) un composant liphophile et
4) un tensioactif,
à condition que lorsque le composant 2) consiste en un mélange de mono-, diglycérides d'acides gras en C₈-C₁₀, ladite composition soit libre de triglycérides d'acides gras en C₆-C₁₂.

2. Composition selon la revendication 1, sous forme d'un préconcentré de microémulsion.

3. Composition selon l'une des revendications 1 ou 2, dans laquelle le second composant comprend un ester d'acide gras en C₆-C₁₀ et de polyéthylèneglycol glycérol ayant de 5 à 10 unités [CH₂-CH₂-O].

4. Composition selon l'une des revendications précédentes, dans laquelle le composant lipophile est sélectionné à partir du groupe composé :
(i) d'huile végétale éthoxylée transestérifiée,
(ii) de mono-, di- et tri-glycérides mélangés,
(iii) de mono- et di-esters d'acide gras et de propylèneglycol, et
(iv) de composés estérifiés d'acide gras et d'alcool primaire.

5. Composition selon l'une des revendications précédentes, comprenant également un ou plusieurs antioxydants sélectionnés à partir du groupe composé de palmitate d'ascorbyle, de butyl hydroxy anisole (BHA), de butyl hydroxy toluène (BHT) et de tocophérole dans des quantités de 0,05 à 1% en poids du poids total de la composition.

6. Composition selon la revendication 5, dans laquelle l'antioxydant présent est l'α-tocophérol.

7. Composition selon l'une des revendications précédentes comprenant la cyclosporine ou un macrolide en une quantité de 1 à 5% en poids de la composition.

8. Composition selon l'une des revendications précédentes, comprenant le second composant en une quantité de 5 à 50%, le composant lipophile en une quantité de 5 à 85% et le tensioactif en une quantité de 5 à 80% en poids du milieu véhicule.

9. Composition selon l'une des revendications précédentes, caractérisée en que les proportions relatives de cyclosporine ou de macrolide, du second composant, du composant lipophile et du tensioactif dans ladite composition sont telles qu'après dilution avec de l'eau à un taux de 1 partie en poids de ladite composition pour 1 à 10 parties en poids d'eau, une microémulsion d'huile dans l'eau ayant une taille de particules inférieure à 200 nm se forme spontanément.

10. Composition selon l'une des revendications précédentes, dans laquelle la cyclosporine est la Cyclosporine A.

11. Composition selon l'une des revendications précédentes, dans laquelle le tensioactif est sélectionné à partir du groupe composé (i) de produits de réaction d'huile végétale naturelle ou hydrogénée et d'oxyde d'éthylène et (ii) d'esters d'acide gras et de polyoxyéthylène sorbitane.

12. Composition selon l'une des revendications précédentes, dans laquelle le taux cyclosporine ou macrolide : second composant : composant lipophile : tensioactif est de 1:0,1 à 10:1 à 10:1 à 10 par rapport au poids.

13. Composition selon l'une des revendications précédentes, dans laquelle un co-tensioactif hydrophile est également présent.

14. Composition selon la revendication 13, dans laquelle le co-tensioactif hydrophile est un copolymère à blocs polyoxyéthylène polyoxypropylène.

15. Composition selon la revendication 13 ou 14, dans laquelle le second composant et le co-tensioactif hydrophile sont combinés selon un taux de 1:0,1 à 5 par rapport au poids.

16. Composition selon l'une des revendications précédentes sous forme d'une unité de dosage.

17. Composition selon l'une des revendications précédentes utilisable en médecine.

18. Composition selon l'une des revendications précédentes pour la fabrication d'un médicament pour le traitement ou la prévention du rejet de transplants d'organes ou de tissus ou de la maladie du greffon contre l'hôte.

19. Utilisation d'une composition selon l'une des revendications 1 à 17 pour la fabrication d'un médicament pour le traitement ou la prévention d'une condition autoimmune ou inflammatoire.

20. Capsule en gélatine molle ou dure comprenant une composition telle que définie dans l'une des revendications 1 à 17.
